(19) Europäisches Patentamt
European Patent Office
Office européen des brevets

(11) **EP 4 365 292 A1**

(12) **EUROPEAN PATENT APPLICATION**

(43) Date of publication:
**08.05.2024 Bulletin 2024/19**

(21) Application number: **23207337.9**

(22) Date of filing: **02.11.2023**

(51) International Patent Classification (IPC):
**C12N 15/115** (2010.01)  **G01N 33/569** (2006.01)
**C12Q 1/02** (2006.01)

(52) Cooperative Patent Classification (CPC):
**C12N 15/115;** C12N 2310/16

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.11.2022 IT 202200022590**

(71) Applicant: **Consiglio per la ricerca in agricoltura e
l'analisi dell'economia agraria
00184 Roma (IT)**

(72) Inventors:
• **CANFORA, Loredana
00184 ROMA(RM) (IT)**

• **MANFREDINI, Andrea
00184 ROMA (RM) (IT)**
• **MALUSA, Eligio
00184 ROMA (RM) (IT)**
• **MOCALI, Stefano
00184 ROMA (RM) (IT)**

(74) Representative: **Ceccarelli, Ilaria et al
Barzanò & Zanardo Roma S.p.A.
Via Piemonte, 26
00187 Roma (IT)**

Remarks:
The complete document including Reference
Table(s) and the Sequence Listing(s) can be
downloaded from the EPO website

(54) **APTAMER FOR THE DETECTION OF THE MICROORGANISM BACILLUS SUBTILIS**

(57) The present invention relates to an aptamer, i.e., a single-stranded DNA or RNA sequence, for the detection of the microorganism *Bacillus subtilis,* wherein said aptamer is capable of acting as a biosensor for detecting the presence of said microorganism, in particular in the soil.

Fig. 3

EP 4 365 292 A1

**Description**

The present invention relates to an aptamer for the detection of the microorganism *Bacillus subtilis.* In particular, the present invention relates to an aptamer, that is, a single-stranded DNA or RNA sequence, for the detection of the microorganism *Bacillus subtilis,* wherein said aptamer is capable of acting as a biosensor to detect the presence of said microorganism, in particular in the soil.

It is well known that the application, in agriculture, of products containing microorganisms is steadily increasing. In fact, the world market stands at around 10 billion USD and 3 billion USD, respectively, for microorganism-based pesticides and biostimulants. The field application of such products requires their registration at the EU and national levels, together with an indication by the manufacturer of various specifications and analytic methods making it possible to trace their destiny in the environment and prove their medium- and long-term effectiveness, aspects that are closely connected with the ability of the microorganism to adapt and persist in the environment (soil).

At present, 22 microbial strains are registered as pesticides, while 4 groups of microorganisms including various species are admitted for registration as microbial biostimulants. Among them, *Bacillus subtilis* is a bacterial species having potential functions of stimulating plant growth or protecting against agrophages following the inoculation thereof into soil.

In this context, the EFSA is working to establish methods useful for evaluating the risk and traceability of microorganisms introduced into soil.

The greatest difficulty in the research and development of molecular markers to be used in soil lies in the fact of being able to identify markers that are species- or strain-specific, i.e., which are capable of discriminating a species, or a strain thereof, from another one in the soil, which represents a heterogeneous and extremely complex matrix. In fact, billions of microorganisms, many of which unknown, reside in a gram of soil.

The methods presently used for species-specific and/or strain-specific traceability of a specific microorganism in soil mainly rely on the use of methods, such as, for example, PCR, which make it possible to identify specific markers (genetic traits), i.e., discriminating factors in the DNA, and to quantify their presence. The success of species- or strain-specific traceability thus depends on the design and optimisation of pairs of primers and probes (additional sequences that increase specificity) capable of recognising, with high specificity and uniqueness, the microorganism being sought and introduced into the soil. In recent years, quantitative PCR has enabled the effectiveness of traceability to be improved by combining the use of species-specific markers, where available, so as also to provide an estimation of the relative abundance of the strains introduced into the soil.

However, in order to apply the known methods, an in-depth knowledge of the target microorganism is necessary; in particular, it is necessary to know its genome, distinctive biochemical features and ecophysiology in order to be able to design markers that are capable of distinguishing it in soil among hundreds of similar species, whose existence is probably not yet known.

Moreover, the known methods for the tracing and/or quantifying the presence of target microorganisms in soil are based on the use of DNA (also of RNA where possible) and, therefore, in a complex matrix such as soil, cannot avoid extractions of environmental DNA, i.e., of all the DNA present in the soil, which often results in the loss of information as well as high costs and a considerable expenditure of man-days of work.

Classic, so-called "culture-dependent" microbiological methods, which are based on the isolation of the target microorganism with appropriate culture media, are used to a small degree. However, such methods only allow quantitative data with low specificity to be obtained. Furthermore, such methods require the use of different selective culture media and subsequent DNA sequencing for the identification of the target microorganism.

Alternative techniques, such as, for example, ELISA or FISH, are likewise laborious and very costly, as they require specific equipment and laboratory know-how.

Furthermore, none of the known techniques mentioned above can be transferred onto mobile devices for *in situ* detection.

In the light of the foregoing, there appears to be an evident need to provide new methods for the traceability of microorganisms, in particular in soil, which are capable of overcoming the disadvantages of the known methods.

The solution according to the present invention fits into this context; it aims to provide a new marker for the specific detection of the microorganism *Bacillus subtilis.*

In particular, according to the present invention, aptamers capable of detecting the microorganism *Bacillus subtilis* in a species-specific manner have surprisingly been found.

It is well known that aptamers (from the Latin *aptus,* i.e. bind, and *mer,* i.e. sequence) are single-stranded DNA or RNA sequences of small dimensions (40-70bp) capable of selectively binding to a given target, be it a simple structure (molecule, protein) or a complex one (genome) or a cell surface. As high-affinity ligands, furthermore, aptamers can be chemically modified to increase their degree of affinity. This latter characteristic makes aptamers similar to antibodies, but, unlike the latter, aptamers are more stable, do not induce immune responses, are capable of being immobilised on inert supports and are not thermolabile. Aptamers can be easily transferred onto a nanoscale and into "lab-on-a-chip" microfluidic systems as opposed to other biomarkers/biosensors that are limited to being applied on a laboratory scale and also entail higher costs and very long application times.

Aptamers have been widely employed in the biomedical realm and, only more recently, in monitoring food or heavy metal contamination (McConnell et al., 2020).

According to the present invention, aptamers capable of specifically recognising the bacterial species 8. *subtilis* for the purpose of monitoring the microorganism within soil have been found for the first time.

An aptamer according to the invention advantageously enables the microorganism to be identified by means of a detection method that is accurate, sensitive, and repeatable, but also easy to apply and with modest costs. Furthermore, the detection of the aforesaid microorganism using an aptamer according to the invention can also be achieved by means of small portable devices, for example biosensors, which enable the application thereof *in situ.*

As described further below in Example 1, for the selection of the species-specific aptamers according to the present invention, first the complete genome of the target strain B. *subtilis PCM/B00105* was sequenced. On the basis of a bioinformatic analysis, a specific region of the genome was identified, on which a pair of primers was designed for the determination of and search for discriminating aptamer candidates for the traceability and monitoring of B. *subtilis PCM/B00105* in soil. The region corresponds to the *pirK* gene. The choice and selection of the gene region on which to design the pair of primers according to the present invention were decisive for the efficiency and success of the selection of candidate aptamers for the traceability of the target bacterium in the soil. According to the present invention, a pair of primers never previously used was designed for the amplification of a random single-stranded library with a length of about 83bp. The primers designed according to the invention are thus unique. The random single-stranded library obtained by amplification was then used for the selection of aptamer candidates for the target strain.

According to the present invention, the known TOGGLE-SELEX (Song et al. 2017) method was used to select candidate aptamers; modifications were introduced to the protocol in order to optimise the selection thereof and adapt the method to the needs of the ongoing analyses, i.e., the selection of aptamer candidates starting from a pure culture of the microorganism. In particular, unlike in the known method, according to the modified protocol use was made of:

- a single strain of *B. subtilis* (PCM B/00105);
- primers specific for *B. subtilis* (PCM B/00105);
- only 4 selection cycles (rounds).

As mentioned above and as described further below in Example 1, for the selection of aptamers it was chosen to use four selection cycles and four aptamers were generated. However, only two aptamers were selected based on their binding affinity for the target microorganism and the stability of the bond. These aptamers, the object of the present invention, are also referred to here as "AptaGir1" (or Aptamer 1) and "AptaGir2" (or Aptamer 2).

The aptamer selection technology used according to the present invention, though challenging, advantageously enabled the identification of a unique genetic sequence of the target microorganism, that is, a characterising and discriminating one, which renders the occurrence of false positives almost impossible.

An aptamer according to the present invention advantageously enables recognition of the target strain at a cellular level without any need to extract nucleic acids, resulting in a considerable lowering of costs compared to the known methods, both in terms of man-hours and in terms of consumable materials. Furthermore, the use of an aptamer according to the invention makes it possible to perform an innovative *in situ* analysis, never carried out in the field of soil microbial inoculant traceability. In fact, the technique for detecting the microorganism B. *subtilis* by means of an aptamer according to the invention is advantageously transferrable onto a mobile device, for example through the use of a biosensor.

Furthermore, whereas the molecular markers most widely used for the traceability of microorganisms require the use of PCR and quantitative PCR in order to trace and quantify, or of sophisticated techniques, the use of an aptamer according to the present invention requires only an extraction of cells.

The possibility that recognition and the binding between an aptamer of the invention and the target organism will occur at the cellular level not only represents an advantage from a technical and economic standpoint, but also allows the use of this technology directly in the field, as the analysis does not provide for a process of extracting nucleic acids. Furthermore, since aptamers are comparable with antibodies, they show a greater sensitivity in recognising the target organism and greater stability in the bond between the aptamer and the cell, also at high temperatures.

Furthermore, an aptamer can be advantageously modified (for example by adding fluorophores which enable the detection of the bond between the aptamer and organism or cell or molecule, also in fluorometric techniques) without any loss of specificity. To this one may add a longer shelf life of aptamers (if compared to antibodies), and a greater activity (i.e., ability to bind to a given target) in different environmental reactions (Kudlak and Wieczerzak 2019), which enhance and favour the use of aptamers in the field of soil microbial inoculant traceability.

Aptamers were conceived in order to be able to recognise molecules of small or large dimensions, until arriving at cells of microscopic organisms (for example bacteria). Aptamers can form complex secondary structures, such as G-quadruplexes, or branched rings, and it is precisely by virtue of this ability of theirs that they can be used in the development of biosensors.

An aptamer according to the present invention is extremely well-suited to being used in environmental monitoring, being chemically stable, easily modifiable, relatively simple to synthesise and biocompatible.

Furthermore, an aptamer according to the invention can be advantageously combined with nanomaterials for the development of detection systems, where the nanomaterials bring complexity and improve sensitivity, enabling the aptamer to be used in optical, electrochemical, and mechanical sensors.

Furthermore, an aptamer according to the present invention can be advantageously used for the development of new sensors for environmental monitoring, in particular of portable systems, i.e., low-cost, simple-to-use sensors for in situ "detection", wherein nanotechnology is combined with microfluidic technologies, making possible the development of a lab-on-a-chip.

The application of aptamers according to the invention, in particular AptaGir1 and AptaGir2, thus fits into this scenario, enabling the detection of *B. subtilis* in soil, by tracing the presence of the bacterium directly in the field, without having to rely on laboratory methods of nucleic acid extraction and PCR amplification.

In particular, an aptamer according to the invention can be used to detect the microorganism contained in microorganism-based products, such as, for example, biostimulants, biofertilizers, or biopesticides, so as to manage the use thereof and the frequency of applications, by monitoring the presence of the target bacterium in the soil to support decision-making in terms of the dosages and times in which to apply the microbial formulation to the soil.

An aptamer according to the invention can also be used to monitor the shelf life of the aforesaid products, guaranteeing their quality and effectiveness over a given time. Furthermore, an aptamer according to the invention can make it possible to evaluate the persistence of the aforesaid products in the soil and to exploit this information to improve their performance. The latter two aspects can also be used to support the documentation to be produced at the time of registering the formulation and used by all bodies tasked with controlling and preventing fraud in the sector of the development and use of microorganism-based formulations.

It is therefore a specific object of the present invention an aptamer comprising the sequence CCTCTTTTGAGAAGGCCGA (SEQ ID NO:7), said aptamer being selected from 5'-CTACCCCTCTTTTGAGAAGGCCGA-3' (SEQ ID NO:3) or 5'-CCTCTTTTGAGAAGGCCGATCACCA-3' (SEQ ID NO:4), preferably 5'-CTACCCCTCTTTTGAGAAGGCCGA-3' (SEQ ID NO:3).

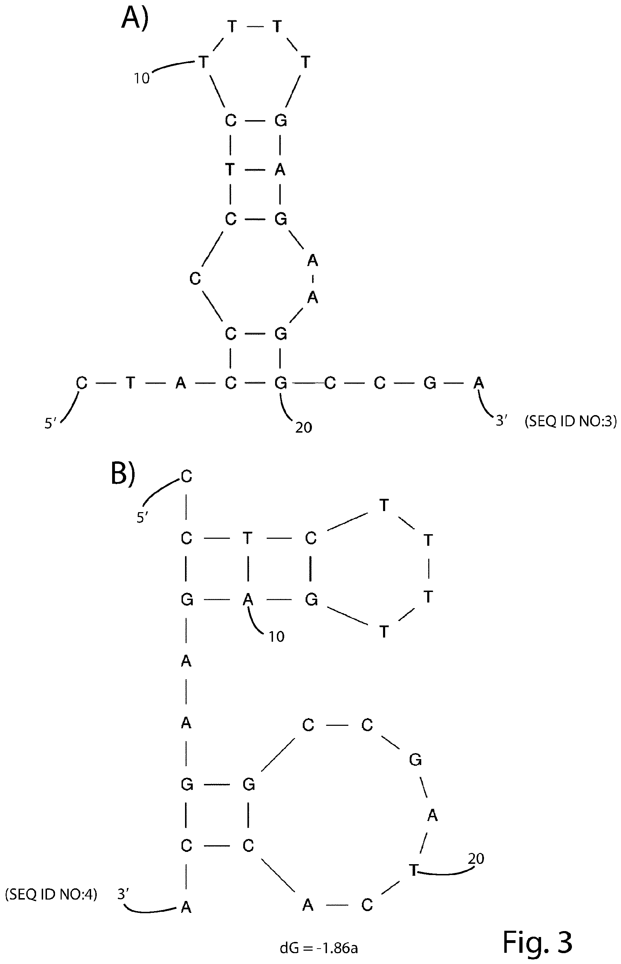

The aptamer according to the invention is suitable for the detection of a microorganism of the species *Bacillus subtilis* in an organic and/or inorganic sample. In particular, the aptamer according to the present invention is a sequence of single-stranded DNA (having the sequence SEQ ID NO:3 or SEQ ID NO:4) capable of assuming the folded conformation shown in Fig.3 and is capable of specifically binding the protein of *Bacillus subtilis,* of about 20 kDa, encoded by the *pyrK* gene.

According to the present invention, said aptamer can be bound to a fluorophore, such as, for example, 6-FAM, VIC, or HEX, or said aptamer can be bound to gold molecules. Binding of the aptamer with gold nanoparticles makes it possible to detect the aptamer and, therefore, to detect and monitor the presence of the species *Bacillus subtilis* using a scanning electron microscope (SEM).

The present invention further relates to a composition for the detection of a microorganism of the species *Bacillus subtilis,* said composition comprising an aptamer as defined above, together with one or more excipients.

The present invention also regards a biosensor for the detection of a microorganism of the species *Bacillus subtilis* in an organic and/or inorganic sample, said biosensor comprising an aptamer as defined above or a composition as defined above.

In particular, biosensor means an analytic device comprising an aptamer as defined above or a composition as defined above.

According to the present invention, said sample can be selected from soil, a composition comprising microorganisms, for example a composition or a formulation comprising microorganisms which is applicable in agriculture (for example the commercial composition BACTIM® SOIL), such as a biostimulating composition or a pesticidal composition, and a plant sample, such as, for example, leaves, roots, or seeds, preferably soil.

According to the present invention, said microorganism of the species *Bacillus subtilis* can be the strain PCM/B00105.

According to one embodiment of the present invention, said biosensor is a chip comprising said aptamer immobilised thereupon, such as, for example, a chip (lab-on-a-chip) based on surface acoustic wave (SAW) technology.

A further object of the present invention is the use of an aptamer as defined above or a composition as defined above for the detection of a microorganism of the species *B. subtilis* in an organic and/or inorganic sample.

As mentioned above, said sample can be selected from soil, a composition comprising microorganisms, for example a composition or a formulation comprising microorganisms which is applicable in agriculture (for example the commercial composition BACTIM® SOIL), such as a biostimulating composition or a pesticidal composition, and a plant sample, such as, for example, leaves, roots, or seeds, preferably soil.

Furthermore, according to the present invention, said microorganism of the species *Bacillus subtilis* can be the strain PCM/B00105.

The present invention further relates to a method for the detection of a microorganism of the species *B. subtilis* in an organic and/or inorganic sample, said method comprising the steps of

a) carrying out sampling of the matrix of interest;

b) extracting cells from the sample with an extractant;

c) placing the extracted cells in contact with an aptamer as defined in any one of claims 1-3 or with a composition as defined in claim 4, so as to make hybridisation occur between said aptamer and said microorganism;

d) detecting the presence of the aptamer-microorganism complex, i.e., detecting the binding that has occurred between the aptamer and the microorganism, in particular by detecting the presence and/or concentration of the aptamer bound to the microorganism by means of a suitable device, such as, for example, a fluorometer (if the aptamer is marked with a fluorophore) or a lab-on-a-chip device, or a scanning electron microscope (for example when the aptamer is marked with gold molecules), wherein said microorganism of the species 8. *subtilis* is present in said sample when the presence of said aptamer-microorganism complex is detected.

Therefore, said aptamer can be marked with a probe and said step d) can consist in detecting the presence of the aptamer-microorganism complex by detecting the presence and/or concentration of the aptamer marked with said probe, by means of a suitable device.

According to the method of the invention, said sample can be selected from soil, a composition comprising microorganisms, for example a composition or a formulation comprising microorganisms which is applicable in agriculture (for example the commercial composition BACTIM® SOIL), such as a biostimulating composition or a pesticidal composition, and a plant sample, such as, for example, leaves, roots, or seeds, preferably soil.

The present invention also relates to a method for identifying an aptamer capable of binding a microorganism of the species *B. subtilis,* said method comprising the use of the following primers:

forward primer: 5'-AAGTGAGCGAAGCGGTTACG-3' (SEQ ID NO:1) (BASU_For)

reverse primer: 5'-AGTTGACATCTGCGATGCTGAT 3' (SEQ ID NO:2).

In particular, said method for identifying an aptamer can comprise the following steps:

a) extracting cells from a bacterial culture of *B. subtilis,* such as, for example, a culture of the strain PCM/B00105, for example through centrifugation and the use of extractants;

b) placing said cells in contact with a single-stranded DNA (ssDNA) library comprising DNA fragments of the pyrK gene of *B. subtilis;*

c) incubating the cells placed in contact with the library for a suitable incubation time so as to make binding occur between any aptamers present in the library and said cells;

d) removing the single-stranded DNA that did not bind to the cells in step c), for example by centrifugation;

e) separating the single-stranded DNA that bound to the cells in step c) from said cells and purifying said DNA;

f) subjecting the single-stranded DNA obtained in step e) to PCR amplification using said primers SEQ ID NO:1 (forward) and SEQ ID NO:2 (reverse), preferably using a high fidelity Taq polymerase;

g) identifying the DNA fragments amplified in said step f), in particular by cloning and sequencing of said fragments;

wherein the DNA fragments amplified in said step f) and identified in said step g) correspond to one or more aptamers capable of binding a microorganism of the species *B. subtilis.*

Therefore, the method according to the present invention makes it possible to identify aptamers capable of binding *B. subtilis* and, subsequently, of selecting therefrom the aptamers having Kd and Bmax values such as to render them suitable for the detection of *B. subtilis* in a sample.

The reverse primer of sequence SEQ ID NO:2 can be modified with biotin at 5'.

According to the method of the invention, in said step f) the following thermal protocol can be used:

I. activation at 95 °C for 5 min

II. denaturation at 95 °C for 30 s

III. denaturation at 56.3 °C for 30 s

IV. extension at 72 °C for 10 s

V. final extension at 72 °C for 5 min,

wherein items II, III and IV are preferably repeated 4 times.

The present invention will now be described by way of non-limiting illustration according to a preferred embodiment thereof, with particular reference to the Example and the figures in the appended drawings, wherein:

- **Figure 1** shows the whole genome sequence (WGS) of B. *subtilis* PCM/B00105; the pyrK region that is object of the selection is indicated with arrows;

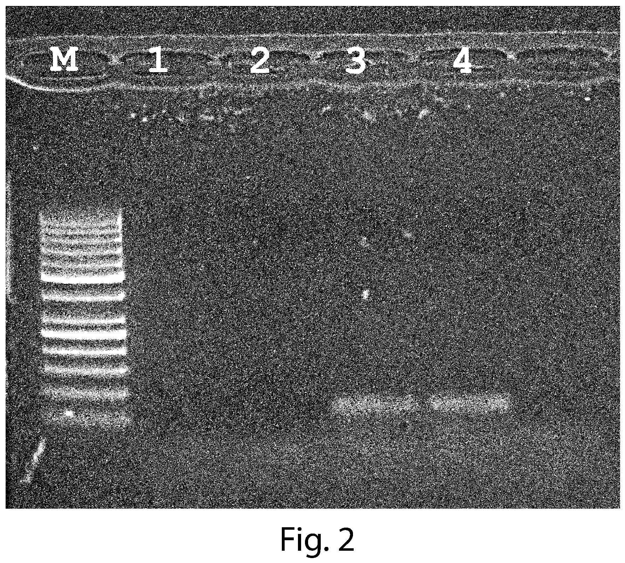

- **Figure 2** shows the result of amplification of the PCR product of the selection cycle. Legend: M marker of the molecular weight 50bp; 1 and 2, negative control; 3 and 4, amplification of the aptamer candidates;
- **Figure 3** shows the folding of the aptamers that were selected and validated according to the present invention, herein also called AptaGir1 **(A)** and AptaGir2 **(B)** (Ref. site for folding: **M. Zuker** Mfold web server for nucleic acid folding and hybridization prediction.

Nucleic Acids Res. 31 (13), 3406-15, (2003) http://www.unafold.org/DNA_form.php DNA Folding Form for 109.115.184.146 **).**

**EXAMPLE 1.** *Selection and validation of aptamers according to the present invention for the detection of B. subtilis.*

The selection of two aptamers specific for *B. subtilis* PCM/B00105 according to the present invention was made in a laboratory. The 7 steps that enabled the development, selection and validation of the aptamer are described below.

1. In silico study and design of the species-specific primers
2. Selection of the aptamers
3. Identification, analysis of the structure and synthesis of the aptamers
4. Validation with pure target microorganisms
5. Validation with a commercial formulation containing *B. subtilis* PCM/B00105
6. Validation in a microcosm in sterile soil inoculated with *B. subtilis* PCM/B00105
7. Validation in an experimental field test on nonsterile soil inoculated with *B. subtilis* PCM/B00105.

### 1. In silico study and design of the species-specific primers

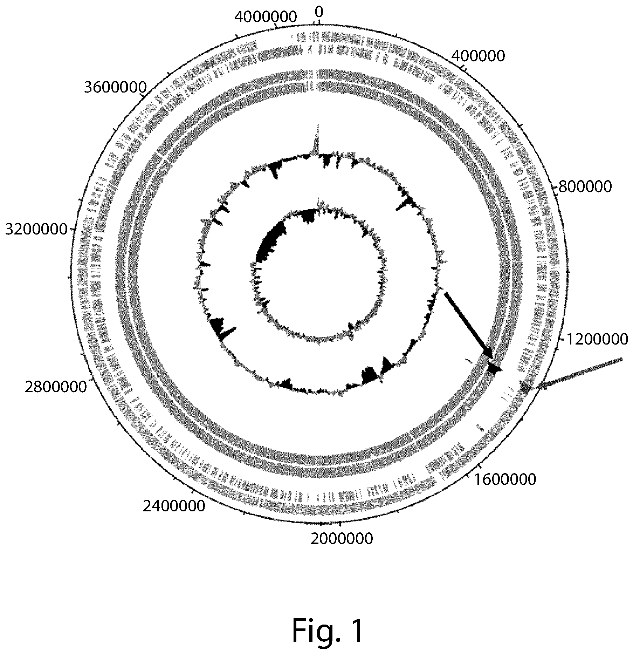

The complete genome of *B. subtilis* PCM/B00105 (strain of *B. subtilis* supplied by the company Intermag; the strain PCM/B00105 is mentioned in patent application WO2017138824 A1 and is deposited with the *Polish Collection of Microorganisms Polish Academy of Sciences in Wroclaw,* under number B/00105) was sequenced by means of the next-generation sequencing approach. A random library of ssDNA with a length of 83bp was designed on the pyrK region (section highlighted in black in Fig. 1). The section was selected as it was conserved and falls in a conserved area of the genome of the species 8. *subtilis.*

### 2. Selection of the aptamers

After the random single-stranded DNA library and the related sequences of the primers of interest had been selected and evaluated, it was necessary to modify the reverse primer with biotin, to be used in the subsequent steps of aptamer purification. Biotin (water soluble B group vitamin) has the ability to form high-affinity bonds with various molecules, including streptavidin, and avidin, which are essential components of many immunoassays. By virtue of this ability, it is increasingly being used in the realm of diagnostics. Purification of the single-stranded DNA library containing the aptamer candidates was carried out using streptavidin-coated magnetic beads. The selection of the aptamer candidates was made possible by virtue of the ability of streptavidin to bind to biotin, present in the reverse primer.

The bacterial strain (*B. subtilis* PCM/B00105) used in the selection and method validation cycle was cultured from a single pure colony made to grow in nutrient broth for 24 h.

The bacterial strain, after a verification of purity and growth, was cultured in broth (NB - nutrient broth) and incubated in an orbital shaker for 24 hours at a temperature of 30°C.

After the incubation period (in the stationary growth phase of the bacterium), a suspension was prepared at a concentration of $1 \times 10^7$ CFU/mL.

The cells of *B. subtilis* were recovered by centrifugation at 18894xg for 10 min; the supernatant was subsequently removed and replaced with PBS (phosphate-buffered saline), at pH 7 (this operation was repeated 3 times).

At the end of the centrifugation, the bacterial pellet was resuspended in 1 mL of binding buffer (consisting of PBS, salmon sperm, bovine serum albumin and Tween-20), to which the ssDNA library was added.

The test tube with *B. subtilis* was incubated at a temperature of 25°C with shaking at 112xg for 1 hour, at the end of which it was subjected to 3 wash cycles (PBS) and centrifugation at 18894xg for 10 minutes to eliminate the part of the ssDNA library not bound to the specific target sequence.

In order to separate the ssDNA from the bacterial cells, the pellet was resuspended in 100 $\mu$L of sterile ultrapure water and subjected to a temperature of 95°C for 10 min and immediately cooled to 4°C for 10 minutes in ice.

The ssDNA in the supernatant was recovered by centrifugation at 18894xg for 10 minutes and ultrafiltered using filter units with dimensions of 50K (50,000 NMWL, or nominal molecular weight limits) to remove the salmon sperm, bovine serum albumin and all the other proteins or interferents that might have remained in the solution.

The purity of the DNA in the filtrate was determined by spectrophotometric analysis, and the quantity was also evaluated by determination of the 260/280 nm ratio.

The DNA elution ratio in the filtrate was calculated by comparing the ssDNA concentration before and after binding with the target.

The recovered ssDNA was subjected to PCR with a primer pair specific for *Bacillus subtilis,* but with the reverse primer modified with biotin.

The sequences of the primers used are the following:

forward primer: 5'-AAGTGAGCGAAGCGGTTACG-3' (SEQ ID NO:1) (BASU_For)

reverse primer: 5'-Biotin AGTTGACATCTGCGATGCTGAT 3' (SEQ ID NO:2) (BASU_Rev_biot)

The following thermal protocol was used:

a. activation at 95 °C for 5 min

b. denaturation at 95 °C for 30 s

c. denaturation at 56.3 °C for 30 s

d. extension at 72 °C for 10 s

e. final extension at 72 °C for 5 min

Items b, c and d were repeated 4 times.

At the end of the amplification reaction, the products obtained were subjected to electrophoresis to evaluate the presence of the product, and subsequently subjected to purification using a specific commercial kit (MinElute PCR Purification Kit, QIAGEN).

In order to separate the ssDNA from the dsDNA, streptavidin-coated magnetic beads were used; 50$\mu$L of magnetic beads and 50$\mu$L of amplified product were placed in contact and the solution was incubated for 30 min at room temperature.

In order to separate the products that did not react, 3 wash cycles were carried out with PBS at pH7 using a magnetic plate and employing a solution containing streptavidin-coated magnetic beads (Pierce™ Streptavidin Magnetic Beads - Thermo Fisher); the final product was eluted in 500$\mu$L of NaOH 200mM and incubated at a temperature of 25°C for 10 minutes.

In order to recover the ssDNA, use was made of filter units with a 10K (10,000 NMWL) mesh. The selection steps described above were repeated four times with the aim of increasing the specificity of the aptamers in the selection process, while at the same time decreasing the amount of aptamer candidates.

Furthermore, the protocol described above was repeated twice to confirm the results obtained.

*3. Identification, analysis of the structure and synthesis of the aptamers*

The ssDNA library containing the aptamers was cloned in order to be able to proceed with Sanger sequencing and identification of the aptamers. Sanger sequencing enabled the identification of 4 aptamers, whose in silico sequences exclusively recognise 8. *subtilis.* The sequences of the four identified aptamers are shown below.

Sequence of aptamer 1 ("AptaGir_1"):
CTACC**CCTCTTTTGAGAAGGCCGA** (SEQ ID NO:3).
Sequence of aptamer 2 ("AptaGir_2"):
**CCTCTTTTGAGAAGGCCGA**TCACCA (SEQ ID NO:4)
Sequence of aptamer 3:
CGATCAGCATCGCAGATGTCA (SEQ ID NO:5)
Sequence of aptamer 4:
CGATCAGCATCGCAGATGTCAAC (SEQ ID NO:6)

The sequences that showed to provide the best performances are the sequences of aptamer 1 (SEQ ID NO:3, or AptaGir1) and aptamer 2 (SEQ ID NO:4, or AptaGir2), illustrated in Fig. 3.

In sequences SEQ ID NO:3 and SEQ ID NO:4, the portion of the sequence in common between the two sequences is highlighted above in bold.

4. *Validation of the method with AptaGir on the pure target microorganism*

The aptamers used for the validation tests were synthesised and associated with FAM labelling to allow their determination by fluorometric analysis. The analytic values obtained with that determination made it possible to evaluate the Kd and Bmax, two parameters useful for assessing the specificity of aptamers.

The values obtained from the fluorometric analysis were processed with a nonlinear regression based on the following formula (as reported in the literature):

$$F = B\_max \times C/((Kd+C))$$

Where:

F represents the detected fluorescence intensity;
Bmax represents the maximum binding intensity;
Kd represents the dissociation constant;
C represents the concentration of the ssDNA aptamer.

Specifically:

Bmax is the maximum specific binding in the same units of Y (RFU). It is the specific binding extrapolated at very high aptamer concentrations; therefore, its value is always greater than any specific binding measured in an experiment;
Kd is the aptamer concentration necessary to obtain semi-maximum binding at equilibrium, expressed in the same units of X (nM).

The higher Bmax is, the higher the binding intensity, while the lower Kd is, the stronger the binding, and it is thus less likely that there will be dissociation. The choice of an aptamer is determined by evaluating both these factors, as described further below. Aptamer 1 (AptaGir) showed to have the best ability to discriminate (and identify), with high sensitivity and specificity compared to other strains.

For the determination of Kd, aptamer concentrations of: 0, 5, 12.5, 25, 50, 125 and 250 nM were used, and to evaluate specificity we used the following strains: *B. cereus* NTC 7464 (BC7464), *B. firmus* (BFK), *B. subtilis* NTC10400 (BS10400), *B. licheniformis* PCM B/00106 (BL106) and *Soilbacillus* sp. (SB_AA_BH_BS).

In order to evaluate the specificity of the aptamers, the following strains of the genus Bacillus were used and compared with the target strain B. *subtilis* PCM B/00105 (BS105): *8. cereus* NTC 7464 (BC7464), *B. firmus* (BFK), *8. subtilis* NTC10400 (BS10400), *B. licheniformis* PCM B/00106 (BL106) and *Soilbacillus* sp. (SB_AA_BH_BS).

The results show a low Kd as regards *B. subtilis* PCM B/00105 with aptamer 1 (AptaGir1) compared with the other strains used and a Bmax close to 1, hence a very high binding affinity.

As regards aptamer 2 (AptaGir2), the latter showed an efficacy comparable to that of aptamer 1. However, aptamer 2 showed a different range of the minimum and maximum concentration necessary to be able to function in recognising and binding to the target.

The minimum and maximum concentration values of Aptagir_1 and Aptagir_2 are shown in Table 1:

**Table 1**

| Species | AptaGir_1 (nM) (Min) | AptaGir_1 ($\mu$M) (Max) | AptaGir_2 (nM) (Min) | AptaGir_2 ($\mu$M) (Max) |
|---|---|---|---|---|
| *B. subtilis* **PCM/B105** | 2.151 | 42 | 0 | 48 |
| *B.licheniformis* **PCM/B106** | 30.01 | 50 | 0 | 100 |
| *B.subtilis* **NTC10400** | 17.97 | 100 | 0 | 100 |
| *P.protegens* | 0 | 0 | 0 | 0 |

The maximum concentration indicated is the maximum concentration under the conditions described above.

The strain *Pseudomonas protegens* is a Gram-negative bacterium used as a negative control.

Aptamer 3 showed a worse Kd than aptamer 1, considered not sufficient, but it nonetheless showed specificity vis-à-vis BS105, compared to that for the other strains.

Aptamer 4 showed, specifically, Kd and Bmax values that were not sufficient and not comparable with those of the other aptamers.

The Bmax and Kd results of AptaGir_1 and AptaGir_2 are shown in Table 2. The codes refer to the strains used.

**Table 2**

|  |  | BSBS105 | BL106 | BS10400 | BC7434 | BFK | SB_AA_ BH_BS |
|---|---|---|---|---|---|---|---|
| AptaGir_1 * | *Kd* | 2.151 | 30.01 | 17.97 | 20.68 | 10.41 | 2.163 |
|  | *Bmax* | 0.995 | 0.7793 | 0.9377 | 1.84 | 0.5896 | 0.5021 |
| AptaGir_2 ** | *Kd* | 3.902 | 29.06 | 9.673 | 0 | 0 | 0 |
|  | *Bmax* | 0.8789 | 9 | 11.48 | 0 | 0 | 0 |
| * Kd and Bmax calculated starting from an aptamer concentration of 0.25 $\mu$M; <br> ** Kd and Bmax calculated starting from an aptamer concentration of 100 $\mu$M. | | | | | | | |

From the results obtained it is possible to deduce that aptamer 1 and aptamer 2 (AptaGir_1 and AptaGir_2) show high specificity and sensitivity.

From the results obtained it is possible to deduce that AptaGir_1, however, is considerably better than AptaGir_2, since the reference concentrations are lower as far as the minimum and maximum thresholds are concerned.

*5. Validation of the method with AptaGir 1 in a microcosm in sterile soil inoculated with B. subtilis PCM/B00105*

The validation test in soil entailed setting up a test in a microcosm (100 mL jar), using three soils that differed in terms of the main physicochemical parameters that most greatly influence the persistence of microorganisms (content of organic substance, pH, texture) and precisely:

- soil originating from Italy (clayey, with an alkaline pH);
- soil originating from Poland (loamy tending towards sandy, with an acidic pH);
- soil originating from Germany (soil with medium texture and a neutral pH).

The soils were appropriately sterilised before the test. The validation test also entailed the development of a solution for extracting the cells from the soil without damaging them, in order to obtain a maximum recovery efficiency. The extraction solution developed is fruit of the authors' inventiveness and is based on the use of a pyrophosphate-based buffer.

Two technical replicas were prepared for each type of soil and inoculated with the strain BS105; a non-inoculated control was also included. The microcosms were incubated at a temperature of 25°C.

In the treated microcosms, 3 inoculations with the strain B. *subtilis* PCM B/00105 were performed 3 days apart at a concentration of $1 \times 10^7$ cells mL$^{-1}$. The duration of the test was 12 days.

At the end of the test, 1 mL of sodium pyrophosphate was added in each microcosm to favour detachment of cells from the soil particles, while subjecting it to shaking for about 1 minute and allowing the suspension to settle for 30 minutes; the supernatant was subsequently collected and divided into three 2 mL test tubes, to which increasing concentrations of aptamer1 (AptaGir_1) (0, 50, 250 nM) were then added.

The results show that in the Italian soil, essentially clayey, no result was obtained, as very few cells were extracted with the pyrophosphate method.

The determination of the strain inoculated into the samples as carried out with the method described above allowed for detecting the presence of *Bacillus subtilis* in loamy-sandy soil (Polish) and soil of medium texture (German), but not in clayey soil (Italian). With the aim of improving and optimising the extraction of cells from clayey soil, Tween 20% was added to the extraction solution used with the pyrophosphate method, which made it possible to determine the presence of B. *subtilis* PCM/B00105 with that type of soil as well.

*6. Validation of the method with AptaGir 1 with a formulation containing 8. subtilis PCM/B00105*

The target strain was monitored and traced in the BACTIM® formulation of the company INTERMAG S.p.a.,

which also contains the strain in question (*B. subtilis* PCM/B00105). Before applying and using AptaGir for the traceability of *B. subtilis* PCM/B00105, the formulation was subjected to an extraction of DNA, suitably amplified with the species-specific primers described in paragraphs 1 & 2, thus confirming the presence of *B. subtilis* PCM/B00105 in the product. Furthermore, a direct culture method was also used to isolate and count the target strain directly on a nutrient substrate. The product contained 1.17E+10 CFU/mL of *8. subtilis* PCM/B00105.

For the fluorometric test for detecting the presence of *B. subtilis* PCM/B001 05 in the BACTIM® formulation, 7 concentrations of AptaGir (from 0 to 250 mM) were used. The BACTIM® formulation was concentrated by centrifugation, and the pellet obtained suitably resuspended in PBS 1x pH 7 and processed according to the protocol described in paragraph 5 to favour the detachment of the cells. The analysis confirmed the presence of BS105 both when performed on the formulation as is (Bactim) and when performed on the DNA extracted from the formulation.

The results of the analyses performed on the formulation are presented in Table 3. In particular, Table 3 shows the Bmax and Kd results for aptamer 1 used for the traceability of *B. subtilis* PCM/B00105 in the Bactim formulation as is.

**Table 3**

|  | **Bactim** |
|---|---|
| *Kd* | 3.243 |
| *Bmax* | 23.74 |

The test on the DNA extracted from the formulation was conducted to have further confirmation of the presence of BS105 in the formulation (data not shown).

*7. Validation of the method with AptaGir 1 on soil obtained from an experimental field test*

The objective of this test was to evaluate the specificity of AptaGir_1 in a real field situation, in the full knowledge of the ubiquitousness of *B. subtilis* spp. in the soil ecosystem, making it possible to verify the ability of the strain to persist in the soil.

Within the framework of the EXCALIBUR project, BACTIM® was applied in various experimental fields. The formulation was applied to a litter composed of apple tree leaves and not directly to the soil, in order to favour the process of degradation thereof. Soil was collected in the points where the litter had been treated with the formulation, three months after the application. Soil samples were also duly taken where the litter had not been treated.

The soil was suitably prepared with sodium pyrophosphate (1% W/V) and Tween 20% in order to favour the detachment of cells from the mineral and organic fraction. The soil thus prepared was subjected to the protocol described in paragraph 5, without detecting the presence of *B. subtilis* PCM/B00105, as emerges from the data shown in Table 4. In particular, Table 4 shows the Bmax and Kd results for aptamer 1, used for the traceability of *B. subtilis* PCM/B00105 in soil treated with Bactim.

**Table 4**

|  | **Control: soil only** | **Soil treated with Bactim** |
|---|---|---|
| *Kd* | 0.9326 | 0.8385 |
| *Bmax* | 4.24E-06 | 2.47E-06 |

This result confirms the specificity of AptaGir_1 for the target strain (it did not give false positives) and, together with the results obtained in paragraph 5, it provides useful indications for environmental monitoring purposes.

*Note: all the strains used in this test are not included in "*ANNEX XL VI LIST OF CLASSIFIED BIOLOGICAL AGENTS (Legislative Decree No 81 of 9 April 2008, updated to October 2021*). Therefore, they require manipulations, to be performed simply according to good laboratory practices.*

The present invention has been described by way of non-limiting illustration, according to the preferred embodiments thereof, but it is to be understood that variations and/or modifications may be introduced by a person skilled in the art without going outside the relevant scope of protection as defined by the appended claims.

**Claims**

1. Aptamer comprising the sequence CCTCTTTTTGAGAAGGCCGA (SEQ ID NO:7), said aptamer being selected from 5'-CTACCCCTCTTTTGAGAAGGCCGA-3' (SEQ ID NO:3) or 5'-CCTCTTTTGAGAAGGCCGATCACCA-3' (SEQ ID NO:4), preferably 5'-CTACCCCTCTTTTGAGAAGGCCGA-3' (SEQ ID NO:3).

2. Aptamer according to claim 1, said aptamer being bound to a fluorophore, such as, for example, 6-FAM, VIC, or HEX.

3. Aptamer according to any one of claims 1-2, said aptamer being bound to gold molecules.

4. Composition for the detection of a microorganism of the species *Bacillus subtilis,* said composition comprising an aptamer as defined in any one of claims 1-3, together with one or more excipients.

5. Biosensor for the detection of a microorganism of the species *Bacillus subtilis* in an organic and/or inorganic sample, said biosensor comprising an aptamer as defined in any one of claims 1-3 or a composition as defined in claim 4.

6. Biosensor according to claim 5, wherein said sample is selected from soil, a composition comprising microorganisms, for example a composition comprising microorganisms which is applicable in agriculture, such as a biostimulating composition or a pesticidal composition, and a plant sample, such as, for example, leaves, roots, or seeds, preferably soil.

7. Biosensor according to any one of claims 5-6, wherein said microorganism of the species of *Bacillus subtilis* is the strain PCM/B00105.

8. Biosensor according to any one of claims 5-7, wherein said biosensor is a chip comprising said aptamer immobilised thereupon, such as, for example, a chip based on surface acoustic wave technology.

9. Use of an aptamer as defined in any one of claims 1-3 or of a composition as defined in claim 4, for the detection of a microorganism of the species *B. subtilis* in an organic and/or inorganic sample.

10. Use according to claim 9, wherein said sample is selected from soil, a composition comprising microorganisms, for example a composition comprising microorganisms which is applicable in agriculture, such as a biostimulating composition or a pesticidal composition, and a plant sample, such as, for example, leaves, roots, or seeds, preferably soil.

11. Use according to any one of claims 9-10, wherein said microorganism of the species *Bacillus subtilis* is the strain PCM/B00105.

12. Method for the detection of a microorganism of the species 8. *subtilis* in an organic and/or inorganic sample, said method comprising the steps of:

   a) carrying out sampling of the matrix of interest;
   b) extracting cells from the sample with an extractant;
   c) placing the extracted cells in contact with an aptamer as defined in any one of claims 1-3 or with a composition as defined in claim 4, so as to make hybridisation occur between said aptamer and said microorganism;
   d) detecting the presence of the aptamer-microorganism complex, wherein said microorganism of the species *B. subtilis* is present in said sample when the presence of said aptamer-microorganism complex is detected.

13. Method according to claim 12, wherein said sample is selected from soil, a composition comprising microorganisms, for example a composition comprising microorganisms which is applicable in agriculture, such as a biostimulating composition or a pesticidal composition, and a plant sample, such as, for example, leaves, roots, or seeds, preferably soil.

14. Method for identifying an aptamer capable of binding a microorganism of the species *B. subtilis,* said method comprising the use of the following primers forward primer: 5'-AAGTGAGCGAAGCGGTTACG-3' (SEQ ID NO:1) reverse primer: 5'-AGTTGACATCTGCGATGCTGAT 3' (SEQ ID NO:2).

15. Method according to claim 14, said method comprising the following steps:

   a) extracting cells from a bacterial culture of *B. subtilis,* for example through centrifugation and the use of extractants;

b) placing said cells in contact with a single-stranded DNA library comprising DNA fragments of the pyrK gene of *B. subtilis;*

c) incubating the cells placed in contact with the library for a suitable incubation time so as to make binding occur between any aptamers present in the library and said cells;

d) removing the single-stranded DNA that did not bind to the cells in step c), for example by centrifugation;

e) separating the single-stranded DNA that bound to the cells in step c) from said cells and purifying said DNA;

f) subjecting the single-stranded DNA obtained in step e) to PCR amplification using said primers SEQ ID NO:1 and SEQ ID NO:2;

g) identifying the DNA fragments amplified in said step f), in particular by cloning and sequencing of said fragments;

wherein the fragments of DNA amplified in said step f) and identified in said step g) correspond to one or more aptamers capable of binding a microorganism of the species *B. subtilis.*

16. Method according to claim 15, wherein in said step f) the following thermal protocol is used:

I. activation at 95 °C for 5 min
II. denaturation at 95 °C for 30 s
III. denaturation at 56.3 °C for 30 s
IV. extension at 72 °C for 10 s
V. final extension at 72 °C for 5 min,

wherein items II, III and IV are preferably repeated 4 times.

Fig. 1

Fig. 2

A)

(SEQ ID NO:3)

B)

(SEQ ID NO:4)

dG = -1.86a

Fig. 3

Europäisches
Patentamt

European
Patent Office

Office européen
des brevets

# EUROPEAN SEARCH REPORT

Application Number

EP 23 20 7337

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Cockrum Seth Edward: "Aptamer Selections Against Bacterial Toxins and Cells", UT Electronic Theses and Dissertations, 4 November 2013 (2013-11-04), pages 1-161, XP093047346, Retrieved from the Internet: URL:http://hdl.handle.net/2152/21911 [retrieved on 2023-05-16] | 14 | INV. C12N15/115 G01N33/569 C12Q1/02 |
| A | * page 67 - page 96 * | 1-13,15, 16 | |
| A | JP 2011 239749 A (JOSHO GAKUEN; KIRIN BREWERY) 1 December 2011 (2011-12-01) * example 5 * | 1-16 | |

**TECHNICAL FIELDS SEARCHED (IPC)**

C12N

The present search report has been drawn up for all claims

| Place of search | Date of completion of the search | Examiner |
|---|---|---|
| The Hague | 20 March 2024 | Romano, Alper |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 20 7337

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2024

| Patent document cited in search report | Publication date | Patent family member(s) | Publication date |
|---|---|---|---|
| JP 2011239749 A | 01-12-2011 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017138824 A1

**Non-patent literature cited in the description**

- *Nucleic Acids Res.,* 2003, vol. 31 (13), 3406-15, http://www.unafold.org/DNA_form.php

- *ANNEX XL VI LIST OF CLASSIFIED BIOLOGICAL AGENTS,* 09 April 2008